Europäisches Patentamt

European Patent Office

Office européen des brevets

(11) Publication number: **0 579 958 A1**

## EUROPEAN PATENT APPLICATION

(21) Application number: 93109794.3

(22) Date of filing: **18.06.93**

(51) Int. Cl.5: **A61K 37/02**, C12P 21/02, //C07K13/00,C12N1/16, C12N1/20,C12N5/00, C12N15/00,(C12P21/02, C12R1:19),(C12P21/02, C12R1:865),(C12P21/02, C12R1:91)

(30) Priority: **19.06.92 JP 161454/92**

(43) Date of publication of application:
**26.01.94 Bulletin 94/04**

(84) Designated Contracting States:
**DE FR GB IT**

(71) Applicant: **Ajinomoto Co., Ltd.**
**15-1, Kyobashi 1-chome, Chuo-ku**
**Tokyo(JP)**

(72) Inventor: **Asano, Takehide**
**14-11, Masago 4-chome,**
**Mihama-ku**
**Chiba-shi, Chiba-ken(JP)**
Inventor: **Kenmochi, Takashi**
**12-36, Fujimi 3-chome**
**Higashimurayama-shi, Tokyo(JP)**
Inventor: **Isono, Kaichi**
**2-14, Nobuto 5-chome,**
**Chuo-ku**
**Chiba-shi, Chiba-ken(JP)**
Inventor: **Mitsui, Akira, c/o Central Res. Lab.**

**Ajinomoto**
**Co., Inc.,**
**No. 1-1, Suzuki-cho,**
**Kawasaki-ku**
**Kawasaki-shi, Kanagawa-ken(JP)**
Inventor: **Hirakawa, Tadashi, c/o Central Res.**
**Lab. Ajinomoto**
**Co., Inc.,**
**No. 1-1, Suzuki-cho,**
**Kawasaki-ku**
**Kawasaki-shi, Kanagawa-ken(JP)**
Inventor: **Hamuro, Junji, c/o Central Res. Lab.**
**Ajinomoto**
**Co., Inc.,**
**No. 1-1, Suzuki-cho,**
**Kawasaki-ku**
**Kawasaki-shi, Kanagawa-ken(JP)**

(74) Representative: **Strehl Schübel-Hopf Groening**
**& Partner**
**Maximilianstrasse 54**
**D-80538 München (DE)**

(54) **A preventive or therapeutic medicine for pancreatitis.**

(57) A polypeptide posssessing a human ADF activity is used for producing a composition for the treatment of pancreatitis.

It may be expected to be clinically applicable as a treatment for acute pancreatitis, infectious pancreatitis, the acute advanced stage of chronic recurrent pancreatitis and acute pancreatitis following surgical therapy, as well as a wide variety of diseases which accompany pancreatic disorders, such as multiple organ failure which accompanies acute pancreatitis, etc.

EP 0 579 958 A1

## FIELD OF THE INVENTION

The present invention relates to the use of a human ADF-polypeptide for producing a new medicament against pancreatitis.

## DESCRIPTION OF THE PRIOR ART

In acute pancreatitis, pancreatic enzymes such as trypsin, chymotrypsin, elastase, kallikrein, phospholipase A2, etc. are activated in the acinar (acine) cells or interstitium of the pancreatic gland due to biliary diseases, alcohol, bacterial infection and other causes, which leads to a chain reaction of disruption of the acinar cells and further activation of pancreatic enzymes, and autolysis of the pancreas thus begins. In more advanced cases, the effects reach the entire body causing serious illness. During these processes, the activated pancreatic enzymes and their products play a major part in the destruction of the pancreas and its surrounding tissue (liver, gallbladder, Pancreas, Vol. 9, 1127, 1984). On the other hand, the activated chymotrypsin, etc. in turn activates xanthine oxidase (XOD), that is a free radical producing enzyme present in pancreatic tissue, which produces a large amount of superoxide which is a free radical, in the pancreas. Said tissue undergoes serious damage due to this superoxide. Protease and free radicals are also produced in large amounts by granulocytes which infiltrate the site of inflammation, thus compounding the damage to the organ (J.Act. Oxyg. Free Rad. Vol. 2, No. 5, 623-629, 1991). In addition, inflammatory local infiltration cells produce active oxygen intermediates due to the effect of various locally produced cytokines (IL-1, IL-6, TNF, etc.), leading to even more serious organ damage.

Incidentally, in the pancreas and in the blood there exist protease inhibitors having the function of suppressing the outbreak of inflammation by inhibiting the activation of pancreatic enzymes, deactivating and removing them. Also, there exist free radical-eliminating enzymes such as glutathione peroxidase, superoxide dismutase, catalase, etc. and antioxidant substances such as $\alpha$-tocopherol, ascorbic acid, CoQ10, etc. in the tissues which eliminate the free radicals produced by xanthine oxidase or granulocytes, defending the body thereby. However, when the invading factors prevail over the biophylaxis factors, damage to the tissue progresses and pancreatitis results.

In addition, although pancreatitis is a benign disease, a 30 % death rate has been reported in cases of acute pancreatitis. The causes of such cases include severe circulatory failure, kidney failure, respiratory failure and cardiac failure, but the majority of deaths result from a condition known as multiple organ failure (MOF) involving a plurality of malfunctions. It has recently become clear that proteases and free radicals are important factors in the progression from acute pancreatitis to multiple organ failure (Igaku no Ayumi, Vol. 156, No. 6, 387-391, 2/9/91).

Modern treatment of acute pancreatitis employs protease inhibitors such as urinastatin, gabexate mesilate, nafamostat mesilate, etc. Nevertheless, though these protease inhibitors experimentally exhibit an excellent effect, their therapeutic effects are inadequate in the clinic, and therefore the development of a drug based on a different mechanism than protease inhibitor has been sought (Igaku no Ayumi, Vol. 156, No. 6, 392-396, 2/9/91).

Thus, a drastic improvement may be expected in the treatment of pancreatitis if it is possible not only to suppress the activity of the proteases, but also to suppress the production of and eliminate free radicals, which are another important cause of pancreatitis. This would also apply to the inhibition outbreak to multiple organ failure. However, at present there does not exist any commercially available medicine for treatment of pancreatitis which eliminates the excessive free radicals produced in the case of pancreatitis and effectively suppresses the outbreak and development thereof.

The object of the present invention is to provide a preventive or therapeutic medicine for pancreatitis which eliminates the excessive free radicals produced in the case of pancreatitis or acute pancreatitis and effectively suppresses the outbreak and development of the disease.

The inventors of the present invention conducted diligent research in order to overcome the above mentioned disadvantages, and surprisingly discovered that a peptide possessing human ADF activity exhibits an excellent effect as a prophylactic treatment for pancreatitis, thus completing the present invention. That is, the present invention relates to the use of a human ADF polypeptide as an effective ingredient for producing a pharmaceutical composition for the prophylaxis or therapeutic treatment of pancreatitis.

In a preferred embodiment the human ADF polypeptide is selected from any of the following polypeptides (a) to (h) :

(a) a polypeptide having the following amino acid sequence :

```
Val Lys Gln Ile Glu Ser Lys Thr Ala Phe Gln Glu Ala Leu Asp Ala
 1               5                   10              15
Ala Gly Asp Lys Leu Val Val Val Asp Phe Ser Ala Thr Trp Cys Gly
         20                  25                  30
Pro Cys Lys Met Ile Lys Pro Phe Phe His Ser Leu Ser Glu Lys Tyr
         35                  40                  45
Ser Asn Val Ile Phe Leu Glu Val Asp Val Asp Asp Cys Gln Asp Val
    50                  55                  60
Ala Ser Glu Cys Glu Val Lys Cys Met Pro Thr Phe Gln Phe Phe Lys
    65              70              75                      80
Lys Gly Gln Lys Val Gly Glu Phe Ser Gly Ala Asn Lys Glu Lys Leu
            85                  90                      95
Glu Ala Thr Ile Asn Glu Leu Val
            100
```

(b) a polypeptide having the following amino acid sequence :

```
Met Val Lys Gln Ile Glu Ser Lys Thr Ala Phe Gln Glu Ala Leu Asp
 1               5                   10              15
Ala Ala Gly Asp Lys Leu Val Val Val Asp Phe Ser Ala Thr Trp Cys
            20                  25                  30
Gly Pro Cys Lys Met Ile Lys Pro Phe Phe His Ser Leu Ser Glu Lys
            35                  40                  45
Tyr Ser Asn Val Ile Phe Leu Glu Val Asp Val Asp Asp Cys Gln Asp
    50                  55                  60
Val Ala Ser Glu Cys Glu Val Lys Cys Met Pro Thr Phe Gln Phe Phe
65                  70                  75                      80
Lys Lys Gly Gln Lys Val Gly Glu Phe Ser Gly Ala Asn Lys Glu Lys
                85                  90                      95
Leu Glu Ala Thr Ile Asn Glu Leu Val
            100                 105
```

(c) a polypeptide, which in respect to (a) and/or (b) is deficient in one or more amino acids;

(d) a polypeptid, in which in respect to (a) and/or (b) one or more amino acids are replaced;

(e) a fusion polypeptide comprising a polypeptide according to (a), (b), (c), or (d) and additional amino acids in which the additionally connected amino acids do not interfere with the biological ADF activity or may be easily eliminated;

(f) a polypeptide, which is an allelic derivative of a polypeptide according to any of (a), (b), (c), (d) or (e);

(g) a polypeptide, in which in the amino acid sequence (a), (b) or (c) additional amino acids are inserted and

(h) a polypeptide having any of the amino acid sequences (a) to (g) which is chemically modified or to which sugar chains are linked.

In another preferred embodiment the ADF polypeptide used for preparing the composition against pancreatitis is produced by gene recombinant techniques.

The existence of the activity of human ADF as an IL-2 receptor-derived factor present in the supernatant from ATL-2 cells was reported in 1985 by Tagaya, et al., and its amino acid sequence and DNA sequence have already been determined, while its production in E. coli has also been reported (Japanese Patent Application Laid Open No. 1-85097. In addition, later analysis of human ADF has resulted in its tentative classification into the family Thioredoxin, which includes oxidation-reduction proteins present in a wide variety of organisms from E. coli to mammals, based on the homology of their amino acid sequences.

The present substance is thus sometimes called thioredoxin by researchers, but as concerns the present invention, it shall be referred to by its traditional name, human ADF.

Human ADF is presently being investigated for use as an anti-inflammatory agent and a radioprotective agent (Japanese Patent Application Laid Open No. 3-204818).

The polypeptide possessing human ADF activity (hereunder abbreviated to "human ADFP") used according to the present invention may be one having the amino acid sequence listed as Sequence Identification No. (2) in the Sequence List, but is not limited to it. That is, as long as it possesses human ADFP activity, it may be a polypeptide with a methionine residue attached to the N-terminus (the one listed as Sequence Identification No. 4), a polypeptide with a substitution in the amino acid sequence effected by chemical modification, base substitution, etc., a polypeptide which lacks a portion of the amino acid sequence, a polypeptide with an insertion of an amino acid residue, or a polypeptide with a sugar chain, etc. on the side chain(s).

However, the preferred polypeptides are the polypeptide listed as Sequence Identification No. 2 composed of 104 amino acids beginning with valine at the N-terminus, and the polypeptide listed as Sequence Identification No. 4 having a structure wherein methionine is attached at the N-terminus.

The human ADFP used according to the present invention may be one produced by any method, and is normally one produced by the method described below.

That is, (1) a method wherein human-derived cells (for example, ATL-2 cells, etc.) are cultured and then purification is made from the culture solution or cell extract by a usual method such as salting out, gel filtration chromatography, ion-exchange chromatography, affinity chromatography, chromatofocusing, reverse phase chromatography, hydrophobic chromatography, etc., to obtain the desired human ADFP (Japanese Patent Application Laid open No. 1-85097, Japanese Patent Application Laid Open No. 62-19532), (2) a method wherein the cDNA or genomic sequences comprising the gene of human ADFP are introduced into host cells of Escherichia coli, Bacillus subtilis, yeast, higher animals or plants using a gene recombination method and the recombinant human ADFP is expressed by the host cell, which is followed by purification using a method such as the one described in (1) (Japanese Patent Application Laid Open No. 1-85097), or (3) a method wherein a peptide having an amino acid sequence listed as Sequence Identification No. 1 or 3 in the Sequence List is synthesized according to a peptide chemical synthesis method.

The human ADFP according to the present invention is capable of both eliminating free radicals such as hydrogen peroxide, etc., and causing refolding to reactivate proteins which have been denatured and deactivated by free radicals. Thus, by administration of human ADFP according to the present invention, it is possible to prevent organ damage due to free radicals. In addition, since ADFP is a human-derived protein, it is not recognized as a foreign substance when administered to the human body, and therefore its toxicity is very low.

The safety of human ADFP according to the present invention has been confirmed by animal experiments.

A preparation of this type, as long as it contains human ADFP as an active ingredient thereof and efficiently achieves the object of the present invention, is not restricted as to form, excipient used or method of administration.

The human ADFP preparation according to the present invention is preferably in the form of an intravenous injection, but any non-intravenous form usually applied to preparations may be used. Appropriate forms generally include parenteral administration forms, particularly injections, and preferably isotonic aqueous solutions or suspensions. These may be prepared prior to use from lyophilized preparations containing a carrier, such as mannitol or cyclodextrin. Such a preparation may be sterilized and/or may contain an auxiliary, for example, an antiseptic, stabilizer, moisturizer and/or emulsifier, solubilizer, or a salt and/or buffer to control osmotic pressure.

The preparation according to the present invention may also contain, as necessary, other agents, and may be produced according to known methods, for example, mixing, dissolution or lyophilization. Further, the active ingredient human ADFP may be contained at a proportion of, for example, about 0.1 - 99.9% by weight, and preferably about 1.0 - 99.0% by weight, or up to 100% by weight in the case of lyophilization. Of course, the ranges are not limited to the above mentioned.

The optimum method of administration and dosage of a preparation according to the present invention may be selected by the physician depending on the condition of the patient, but in normal cases a single dosage of approximately 0.01 - 1000 mg, and preferably 0.1 - 100 mg one or more times a day may be given to persons with a body weight of about 50-70 kg.

A more detailed explanation of the present invention is given below with reference to the Examples.

Examples

[Example 1]

Human ADFP-induced suppression of serum amylase release accompanying caerulein pancreatitis.

Rats (Wistar, male, body weight 200-250 g, purchased from Shizuoka Laboratory Animals) were subcutaneously injected with caerulein (150 $\mu$g/kg, ceosnine, KYOWA HAKKO KOGYO CO., LTD.) to induce acute edematous pancreatitis. The rats in the human ADFP-administered group were each injected with a solution of 100 $\mu$g of recombinant human ADFP in 0.25 ml of a phosphate buffer solution (pH 7.4) via the penile vein, immediately after injection with caerulein and 24 hours thereafter. Also, the control group was injected with physiological saline instead of human ADFP. The recombinant human ADFP was prepared following the method described in Japanese Patent Application Laid Open No. 1-85097.

The serum amylase was measured in each group in the following manner. That is, 12 hours and 24 hours after inducement of pancreatitis, blood was taken, and the serum was separated by centrifugation at 3000 rpm for 20 minutes, and measurement was made according to an enzyme method using a Merkotest Amylase Kit for measurement of $\alpha$-amylase activity.

The results are shown in Table 1.

Table 1

| Group | Number of Individuals | Serum amylase level (IU/l) | |
|---|---|---|---|
| | | After 12 hrs | After 24 hrs |
| Control group | 3 | 10450±3041 | 6397±83.8 |
| Recombinant human ADFP-administered group | 3 | 6596±478 | 3627±523 |

As shown in Table 1, administration of recombinant human ADFP resulted in a significant statistical decrease in the blood amylase levels to 63 % compared with amylase levels of the control group at 12 hours after inducement of pancreatitis, and further to 57 % compared with that of the control group at 24 hours thereafter. This means that the release of amylase into the blood accompanying pancreatitis was suppressed by administration of recombinant human ADFP.

[Example 2]

Human ADFP-induced suppression of serum lipase release accompanying caerulein pancreatitis.

Rats (Wistar, male, body weight 200-250 g, purchased from Shizuoka Laboratory Animals) were subcutaneously injected with caerulein (150 $\mu$g/kg, ceosnine, Kyowa Hakko Co.) to induce acute edematous pancreatitis. The rats in the human ADFP-administered group were each injected with a solution of 100 $\mu$g of recombinant human ADFP in 0.25 ml of a phosphate buffer solution (pH 7.4) via the penile vein, immediately after injection with caerulein and 24 hours thereafter. Also, the control group was injected with saline instead of human ADFP.

The serum lipase was measured in each group in the following manner. That is, 12 hours after inducement of pancreatitis, blood was taken, and the serum was separated by centrifugation at 3000 rpm for 20 minutes, and measurement was made based on the enzyme reaction rate with 1,2-dilinoleoyl glycerol as the substrate (UV rate method).

The results are shown in Table 2.

5

Table 2

| Group | Number of Individuals | Serum lipase level (IU/l) |
|---|---|---|
| Control group | 3 | 88.0±48.1 |
| Recombinant human ADFP-administered group | 3 | 40.0±29.7 |

As shown in Table 2, administration of recombinant human ADFP resulted in a decrease in the blood lipase levels to 45% compared with lipase levels of the control group at 12 hours after inducement of pancreatitis. This means that the release of lipase into the blood accompanying pancreatitis was significantly suppressed by administration of recombinant human ADFP.

[Example 3]

Human ADFP-induced suppression of serum amylase release accompanying infectious pancreatitis.

Rats (Wistar, male, body weight 200-250 g, purchased from Shizuoka Laboratory Animals) were opened at the abdomen under ether anesthesia, and 0.1 ml of a mixed solution of dog bile and dog appendeal contents washing solution was injected into the pancreatic capsule to induce localized infectious pancreatitis. The rats in the human ADFP-administered group were each injected with a solution of 100 $\mu$g of recombinant human ADFP in 0.25 ml of a phosphate buffer solution (pH 7.4) via the penile vein, immediately after inducement of infectious pancreatitis and 24 hours thereafter. Also, the control group was injected with saline instead of human ADFP.

The serum amylase was measured in each group in the following manner. That is, one week after inducement of pancreatitis, blood was taken, and the serum was separated by centrifugation at 3000 rpm for 20 minutes, and measurement was made according to an enzyme method using a Merkotest Amylase Kit for measurement of $\alpha$-amylase activity.

The results are shown in Table 3.

Table 3

| Group | Number of Individuals | Serum amylase level (IU/l) |
|---|---|---|
| Control group | 3 | 14167±4030 |
| Recombinant human ADFP-administered group | 3 | 1980±787 |

As shown in Table 3, administration of recombinant human ADFP resulted in a statistically significant decrease in the blood amylase levels at one week after inducement of pancreatitis. This means that the release of amylase into the blood accompanying pancreatitis was suppressed by administration of recombinant human ADFP.

[Example 4]

Human ADFP-induced protection of organism against infectious pancreatitis

Rats (Wistar, male, body weight 200-250 g, purchased from Shizuoka Laboratory Animals) were opened at the abdomen under ether anesthesia, and 0.1 ml of a mixed solution of dog bile and dog appendeal contents washing solution was injected into the pancreatic capsule to induce localized infectious pancreatitis. The rats in the human ADFP-administered group were each injected with 100 $\mu$g of a solution of 100 $\mu$g of recombinant human ADFP in 0.25 ml of a phosphate buffer solution (pH 7.4) via the penile vein, immediately after inducement of infectious pancreatitis and 24 hours thereafter. Also, the control group was injected with physiological saline instead of human ADFP. The sequential variation in the survival rate of each group after inducement of pancreatitis is shown in Table 4.

Table 4

| | Number of Individuals | Survival rate (%) | | | | | | |
|---|---|---|---|---|---|---|---|---|
| | | after 1 day | after 2 days | after 3 days | after 4 days | after 5 days | after 6 days | after 7 days |
| Control group | 15 | 93.3 | 86.7 | 60.0 | 46.7 | 40.0 | 33.3 | 26.7 |
| Recombinant human ADFP-administered group | 8 | 100 | 100 | 100 | 100 | 87.5 | 87.5 | 87.5 |

As shown in Table 4, the infectious pancreatitis was fatal, with only 26.7% of the control group surviving at 7 days after inducement of pancreatitis. However, the survival rate of the recombinant human ADFP-administered group at 7 days thereafter was 87.5%, a significant improvement over the control group.

[Example 5]

Human ADFP-induced protection of tissue against infectious pancreatitis

Rats (Wistar, male, body weight 200-250 g, purchased from Shizuoka Laboratory Animals) were opened at the abdomen under ether anesthesia, and 0.1 ml of a mixed solution of dog bile and dog appendeal contents washing solution was injected into the pancreatic capsule to induce localized infectious pancreatitis. The rats in the human ADFP-administered group were each injected with a solution of 100 $\mu$g of recombinant human ADFP in 0.25 ml of a phosphate buffer solution (pH 7.4) via the penile vein, immediately after inducement of infectious pancreatitis and 24 hours thereafter. Also, the control group was injected with saline instead of human ADFP.

One week after inducement of pancreatitis the pancreas' were removed, and the tissue thereof was cut into sections. The tissue photographs of each of the groups were observed for the frequency of histological signs such as edema bleeding and necrosis.

The results are shown in Table 5.

Table 5

| Frequency of histological signs (%) | | | | |
|---|---|---|---|---|
| | Number of Individuals | edema | Bleeding | Necrosis |
| Control group | 4 | 75 | 75 | 100 |
| Recombinant human ADFP-administered group | 3 | 67 | 33 | 33 |

As shown in Table 5, a high frequency of tissue damage of 75% edema, 75% bleeding and 100% necrosis was observed in the control group, while it was suppressed to a comparatively low frequency of 67%, 33% and 33%, respectively, in the recombinant human ADFP-administered group.

The results of the above mentioned Examples indicate the following.

The pancreatic enzymes amylase and lipase are known to be released into the blood upon outbreak of pancreatitis. Therefore, if the blood concentration of these enzymes are suppressed by a certain drug, it follows that the pancreatitis will also be suppressed. As shown in Examples 1, 2 and 3, the levels of amylase or lipase released into the blood due to caerulein pancreatitis or infectious pancreatitis were suppressed to low levels by administration of recombinant human ADFP, providing biochemical evidence of the inhibiting effect of recombinant human ADFP against pancreatitis. Further, as shown in Example 4, it was confirmed that recombinant human ADFP possesses organism-protecting effect against fatal infectious pancreatitis. Also, the organism-protecting effect of recombinant human ADFP was histologically confirmed, as shown by Example 5.

[Effect of the Invention]

It was clearly shown that human ADFP according to the present invention possesses an excellent organism-protecting effect against pancreatitis. Therefore, it may be expected to be clinically applicable as a treatment for acute pancreatitis, infectious pancreatitis, the acute advanced stage of chronic recurrent pancreatitis and acute pancreatitis following surgical therapy, as well as a wide variety of diseases which accompany pancreatic disorders, such as multiple organ failure which accompanies acute pancreatitis, etc.

SEQUENCE LISTINGS

GENERAL INFORMATION:

APPLICANT:
Name:          AJINOMOTO CO.; Inc.
Street:        No. 15-1 Kyobashi 1-chome, Chuo-ku
City:          Tokyo
Country:       Japan
Postal Code: 104

TITLE OF INVENTION: A preventive or therapeutic medicine for
                    pancreatitis

NUMBER OF SEQUENCES: 4

COMPUTER READABLE FORM:
Medium Type:       Floppy disc
Computer:          IBM PC compatible
Operating System: MS-DOS 5.0

INFORMATION FOR SEQ ID NO:1:

SEQUENCE CHARACTERISTICS:
     Length:    104 amino acids
     TYPE:      amino acid
     TOPOLOGY: linear

MOLECULE TYPE: protein

SEQUENCE DESCRIPTION: SEQ ID NO:1:

Val Lys Gln Ile Glu Ser Lys Thr Ala Phe Gln Glu Ala Leu Asp Ala
 1               5                  10                  15

Ala Gly Asp Lys Leu Val Val Val Asp Phe Ser Ala Thr Trp Cys Gly
             20                  25                  30

Pro Cys Lys Met Ile Lys Pro Phe Phe His Ser Leu Ser Glu Lys Tyr
         35                  40                  45

Ser Asn Val Ile Phe Leu Glu Val Asp Val Asp Asp Cys Gln Asp Val
         50                  55                  60

Ala Ser Glu Cys Glu Val Lys Cys Met Pro Thr Phe Gln Phe Phe Lys
 65                  70                  75                  80

Lys Gly Gln Lys Val Gly Glu Phe Ser Gly Ala Asn Lys Glu Lys Leu
                 85                  90                  95

Glu Ala Thr Ile Asn Glu Leu Val
                100

9

INFORMATION FOR SEQ ID NO:2:

SEQUENCE CHARACTERISTICS:
        LENGTH:    105 amino acids
        TYPE:      amino acid
        TOPOLOGY: linear

MOLECULE TYPE: protein

SEQUENCE DESCRIPTION: SEQ ID NO:2:

```
Met Val Lys Gln Ile Glu Ser Lys Thr Ala Phe Gln Glu Ala Leu Asp
 1               5                  10                  15

Ala Ala Gly Asp Lys Leu Val Val Asp Phe Ser Ala Thr Trp Cys
            20                  25                  30

Gly Pro Cys Lys Met Ile Lys Pro Phe Phe His Ser Leu Ser Glu Lys
            35                  40                  45

Tyr Ser Asn Val Ile Phe Leu Glu Val Asp Val Asp Asp Cys Gln Asp
        50                  55                  60

Val Ala Ser Glu Cys Glu Val Lys Cys Met Pro Thr Phe Gln Phe Phe
 65                  70                  75                  80

Lys Lys Gly Gln Lys Val Gly Glu Phe Ser Gly Ala Asn Lys Glu Lys
                85                  90                  95

Leu Glu Ala Thr Ile Asn Glu Leu Val
            100                 105
```

INFORMATION FOR SEQ ID NO:3:

SEQUENCE CHARACTERISTICS:
     LENGTH:        312 base pairs
     TYPE:          nucleic acid
     STRANDEDNESS: double
     TOPOLOGY:     linear

HYPOTHETICAL: NO

ANTI-SENSE: NO

ORIGINAL SOURCE:
     (A) ORGANISM: Homo sapiens
     (B) STRAIN: ATL-2

FEATURE:
     (A) NAME/KEY: CDS
     (B) LOCATION: 1..312

SEQUENCE DESCRIPTION: SEQ ID NO:3:

```
GTG AAG CAG ATC GAG AGC AAG ACT GCT TTT CAG GAA GCC TTG GAC GCT        48
Val Lys Gln Ile Glu Ser Lys Thr Ala Phe Gln Glu Ala Leu Asp Ala
 1               5                  10                  15

GCA GGT GAT AAA CTT GTA GTA GTT GAC TTC TCA GCC ACG TGG TGT GGG        96
Ala Gly Asp Lys Leu Val Val Val Asp Phe Ser Ala Thr Trp Cys Gly
             20                  25                  30

CCT TGC AAA ATG ATC AAG CCT TTC TTT CAT TCC CTC TCT GAA AAG TAT       144
Pro Cys Lys Met Ile Lys Pro Phe Phe His Ser Leu Ser Glu Lys Tyr
         35                  40                  45

TCC AAC GTG ATA TTC CTT GAA GTA GAT GTG GAT GAC TGT CAG GAT GTT       192
Ser Asn Val Ile Phe Leu Glu Val Asp Val Asp Asp Cys Gln Asp Val
     50                  55                  60

GCT TCA GAG TGT GAA GTC AAA TGC ATG CCA ACA TTC CAG TTT TTT AAG       240
Ala Ser Glu Cys Glu Val Lys Cys Met Pro Thr Phe Gln Phe Phe Lys
 65                  70                  75                  80

AAG GGA CAA AAG GTG GGT GAA TTT TCT GGA GCC AAT AAG GAA AAG CTT       288
Lys Gly Gln Lys Val Gly Glu Phe Ser Gly Ala Asn Lys Glu Lys Leu
                 85                  90                  95

GAA GCC ACC ATT AAT GAA TTA GTC                                       312
Glu Ala Thr Ile Asn Glu Leu Val
                100
```

11

INFORMATION FOR SEQ ID NO:4:

SEQUENCE CHARACTERISTICS:
        LENGTH:        315 base pairs
        TYPE:          nucleic acid
        STRANDEDNESS: double
        TOPOLOGY:      linear

HYPOTHETICAL: NO

ANTI-SENSE: NO

ORIGINAL SOURCE:
        ORGANISM: Homo sapiens
        STRAIN: ATL-2

FEATURE:
        NAME/KEY: CDS
        LOCATION: 1..315

SEQUENCE DESCRIPTION: SEQ ID NO:4:

```
ATG GTG AAG CAG ATC GAG AGC AAG ACT GCT TTT CAG GAA GCC TTG GAC          48
Met Val Lys Gln Ile Glu Ser Lys Thr Ala Phe Gln Glu Ala Leu Asp
 1               5                  10                  15

GCT GCA GGT GAT AAA CTT GTA GTA GTT GAC TTC TCA GCC ACG TGG TGT          96
Ala Ala Gly Asp Lys Leu Val Val Val Asp Phe Ser Ala Thr Trp Cys
            20                  25                  30

GGG CCT TGC AAA ATG ATC AAG CCT TTC TTT CAT TCC CTC TCT GAA AAG         144
Gly Pro Cys Lys Met Ile Lys Pro Phe Phe His Ser Leu Ser Glu Lys
        35                  40                  45

TAT TCC AAC GTG ATA TTC CTT GAA GTA GAT GTG GAT GAC TGT CAG GAT         192
Tyr Ser Asn Val Ile Phe Leu Glu Val Asp Val Asp Asp Cys Gln Asp
        50                  55                  60

GTT GCT TCA GAG TGT GAA GTC AAA TGC ATG CCA ACA TTC CAG TTT TTT         240
Val Ala Ser Glu Cys Glu Val Lys Cys Met Pro Thr Phe Gln Phe Phe
 65                  70                  75                  80

AAG AAG GGA CAA AAG GTG GGT GAA TTT TCT GGA GCC AAT AAG GAA AAG         288
Lys Lys Gly Gln Lys Val Gly Glu Phe Ser Gly Ala Asn Lys Glu Lys
                85                  90                  95

CTT GAA GCC ACC ATT AAT GAA TTA GTC                                     315
Leu Glu Ala Thr Ile Asn Glu Leu Val
            100                 105
```

## Claims

1. Use of a polypeptide having human ADF activity as an effective ingredient for preparing a pharmaceutical composition for the prophylaxis or therapeutic treatment of pancreatitis.

2. The use of a polypeptide having human ADF activity according to claim 1, wherein said polypeptide is selected from any of the following polypeptides (a) to (h) :
        (a) a polypeptide having the following amino acid sequence:

```
Val Lys Gln Ile Glu Ser Lys Thr Ala Phe Gln Glu Ala Leu Asp Ala
 1               5                   10                  15
Ala Gly Asp Lys Leu Val Val Val Asp Phe Ser Ala Thr Trp Cys Gly
            20                  25                  30
Pro Cys Lys Met Ile Lys Pro Phe Phe His Ser Leu Ser Glu Lys Tyr
        35                  40                  45
Ser Asn Val Ile Phe Leu Glu Val Asp Val Asp Asp Cys Gln Asp Val
    50                  55                  60
Ala Ser Glu Cys Glu Val Lys Cys Met Pro Thr Phe Gln Phe Phe Lys
65                  70                  75                  80
Lys Gly Gln Lys Val Gly Glu Phe Ser Gly Ala Asn Lys Glu Lys Leu
            85                  90                  95
Glu Ala Thr Ile Asn Glu Leu Val
            100
```

(b) a polypeptide, which in respect to (a) has a methionin residue attached to the 5'terminus thereof;

(c) a polypeptide, which in respect to (a) and/or (b) is deficient in one or more amino acids;

(d) a polypeptide, in which in respect to (a) and/or (b) one or more amino acids are replaced;

(e) a fusion polypeptide comprising a polypeptide according to (a), (b), (c), or (d) and additional amino acids in which the additionally connected amino acids do not interfere with the biological ADF activity or may be easily eliminated;

(f) a polypeptide, which is an allelic derivative of a polypeptide according to any of (a), (b), (c), (d) or (e);

(g) a polypeptide, in which in the amino acid sequence (a), (b) or (c) additional amino acids are inserted and

(h) a polypeptide having any of the amino acid sequences (a) to (g) which is chemically modified or to which sugar chains are linked.

3. The use of a polypeptide according to the claims 1 or 2 wherein the polypeptide possessing human ADF activity is produced by gene recombinant techniques.

4. The use according to any of the claims 1 to 3, wherein the ADF polypeptide used as an effective ingredient is produced in E. coli.

5. The use of a polypeptide according to any of the preceeding claims, wherein the pharmaceutical composition contains additional auxiliary components.

## DOCUMENTS CONSIDERED TO BE RELEVANT

EP 93109794.3

| Category | Citation of document with indication, where appropriate, of relevant passages | Relevant to claim | CLASSIFICATION OF THE APPLICATION (Int. Cl.5) |
|---|---|---|---|
| D,A | PATENT ABSTRACTS OF JAPAN, unexamined applications, C section, vol. 15, no. 474, December 03, 1991 THE PATENT OFFICE JAPANESE GOVERNMENT page 47 C 890 * No. 3-204 818 (AJINOMOTO CO. INC.) * -- | 1-5 | A 61 K 37/02 /C 12 P 21/02 C 07 K 13/00 C 12 N 1/16 C 12 N 1/20 C 12 N 5/00 C 12 N 15/00 (C 12 P 21/02, C 12 R 1:19) (C 12 P 21/02, |
| D,A | PATENT ABSTRACTS OF JAPAN, unexamined applications, C section, vol. 13, no. 290, July 05, 1989 THE PATENT OFFICE JAPANESE GOVERNMENT page 87 C 614 * No. 1-85 097 (AJINOMOTO CO. INC.) * -- | 1-4 | C 12 R 1:865) (C 12 P 21/02, C 12 R 1:91) |
| D,A | PATENT ABSTRACTS OF JAPAN, unexamined applications, C section, vol. 11, no. 193, June 20, 1987 THE PATENT OFFICE JAPANESE GOVERNMENT page 155 C 430 * No. 62-19 532 (AJINOMOTO CO. INC.) * -- | 1-4 | **TECHNICAL FIELDS SEARCHED (Int. Cl.5)**<br><br>A 61 K<br>C 07 K<br>C 12 N<br>C 12 P |
| A | PATENT ABSTRACTS OF JAPAN, unexamined applications, C section, vol. 11, no. 193, June 20, 1987 THE PATENT OFFICE JAPANESE GOVERNMENT page 155 C 430 * No. 62-19 533 (AJINOMOTO Co. INC.) * -- | 1-4 | |

The present search report has been drawn up for all claims

| Place of search | Date of completion of the search | Examiner |
|---|---|---|
| VIENNA | 06-09-1993 | SCHNASS |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | EP - A - 0 543 240<br>(MOCHIDA PHARMACEUTICAL CO.)<br>  * Claims 1-7,21 * | 1-5 | |
| A | GB - A - 2 125 799<br>(SANDOZ LTD.)<br>  * Claims 1,7-10 * | 1-5 | |

**TECHNICAL FIELDS SEARCHED (Int. Cl.5)**

The present search report has been drawn up for all claims

| Place of search | Date of completion of the search | Examiner |
|---|---|---|
| VIENNA | 06-09-1993 | SCHNASS |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)